Europäisches Patentamt

European Patent Office

Office européen des brevets

(11) Publication number: **0 279 484 B1**

# EUROPEAN PATENT SPECIFICATION

(45) Date of publication of patent specification: **03.06.92**   (51) Int. Cl.⁵: **A61F 2/20**

(21) Application number: **88200217.3**

(22) Date of filing: **08.02.88**

(54) Phonation valve for laryngectomy patients.

(30) Priority: **10.02.87 IT 1931387**

(43) Date of publication of application:
**24.08.88 Bulletin  88/34**

④⑤ Pubication of the grant of the patent:
**03.06.92 Bulletin  92/23**

(84) Designated Contracting States:
**AT BE CH DE ES FR GB GR IT LI LU NL SE**

(56) References cited:
**DE-A- 2 848 592**
**DE-A- 3 211 126**
**FR-A- 2 509 605**
**US-A- 4 060 856**
**US-A- 4 494 252**

(73) Proprietor: **Cassani, Giancarlo**
**Via dei Cedri, 6 Villaggio Residenziale**
**I-20065 Inzago Milano(IT)**

(72) Inventor: **Cassani, Giancarlo**
**Via dei Cedri, 6 Villaggio Residenziale**
**I-20065 Inzago Milano(IT)**

(74) Representative: **Riccardi, Sergio**
**Riccardi & Co. Via Macedonio Melloni, 32**
**I-20129 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a special phonation valve which restores respiration and phonation at levels very similar to normal in patients who have undergone total laryngectomy.

With current techniques, it is known that two separate tracts are created in total laryngectomy patients in order to permit deglutition and respiration.

This creates a situation with acute psychological implications since phonation, which is indispensable for correct enunciation, becomes very approximate and, in addition, depends on the individual's ability to learn how to be a ventriloquist.

In fact, the deglutition tract is the only one that follows the normal course while respiration passes through the stump of the trachea left after the removal of the larynx for which an artificial opening in the skin of the throat has been created by means of a tracheostoma.

FR-A-2 509 605 disclosed a larynx prosthesis consisting of a hollow cylinder with a conical lid which is closed by a valve controlled by a blade spring and having an orthogonal tube fixed to the tracheostoma. This device obliges the patient to keep for ever the tracheostoma open and the blade spring is subject to wear and jamming problems, while it would be highly desirable to have a device without any movable part.

All other known prior art devices have the same problems, and more particularly all require that the patient keeps an open tracheostoma for the entire life.

These extremely serious problems, impairing the psychology and the quality of life of the patients, are solved by a phonation valve having the features recited in the characterizing clause of Claim 1. Further features of the valve are recited in the dependent claims.

The invention as claimed in practice permits the return to a situation which is as close as possible to the normal situation, reincorporating the phonation and deglutition functions at the level of the lower larynx, closing the tracheostoma or at least making use of the tracheostoma for respiration not strictly and/or continuously necessary.

The valve as claimed permits the food to pass into the hypopharynx and then into the oesophagus and the stomach, providing protection in the form of a head for the trachea which lies beneath it, stopping food and liquids from entering the trachea while still permitting the free passage of air from the nose and the mouth to the trachea and viceversa.

The protective function is performed by a mushroom-shaped cap located above the actual valve, through which the air inspired and expired passes.

Since the tracheostoma is no longer used for respiration, it can be closed by a cannula which has a plugged frontal section and a window in the anteriad portion to permit free internal passage of the air. However, it would be preferable to keep the tracheostoma, in order to solve any problems which could arise with the valve of this claim with the passage of time.

It is clear from what has been stated above that the aforesaid valve is an original concept which will permit the patient to lead an almost normal life, eliminating all the anxieties deriving from the fact that the patient has a large frontal aperture in direct contact with the trachea which produces difficulties in being accepted in society, psychological and practical problems of various kinds, the most obvious of which being linked with a slow and ugly way of speaking and difficulty in speaking, apart from the impossibility of taking a shower or a bath, or riding a motorbike etc.

In practice the aforementioned phonation valve can have different forms all falling within the scope of this invention as defined by the appended claims. Embodiments of the invention will be described herinafter in detail, purely as an example and without in any way limiting the scope thereof, supported by the attached illustrative drawings in which:

Figure 1 is a diagram of a patient who has undergone a laryngectomy according to the traditional method;

Figure 2 is a diagram of a patient who has undergone a laryngectomy and who has been fitted with a phonation valve of the invention as claimed;

Figure 3 shows a diagram of a patient who has been fitted with a phonation valve of the invention as claimed and, as a consequence, with the elimination of the tracheostoma;

Figure 4 is a perspective view of a typical cannula used in laryngectomy operations, provided with a window in the anteriad position, which carries air from the lungs to the face of the trachea onto which the phonation valve has been fitted.

Figure 5 is a perspective view of the phonation valve of the invention as claimed, showing the collar provided with slots for the infiltration of the connective tissue and the slots in the drum through which the air passes during respiration.

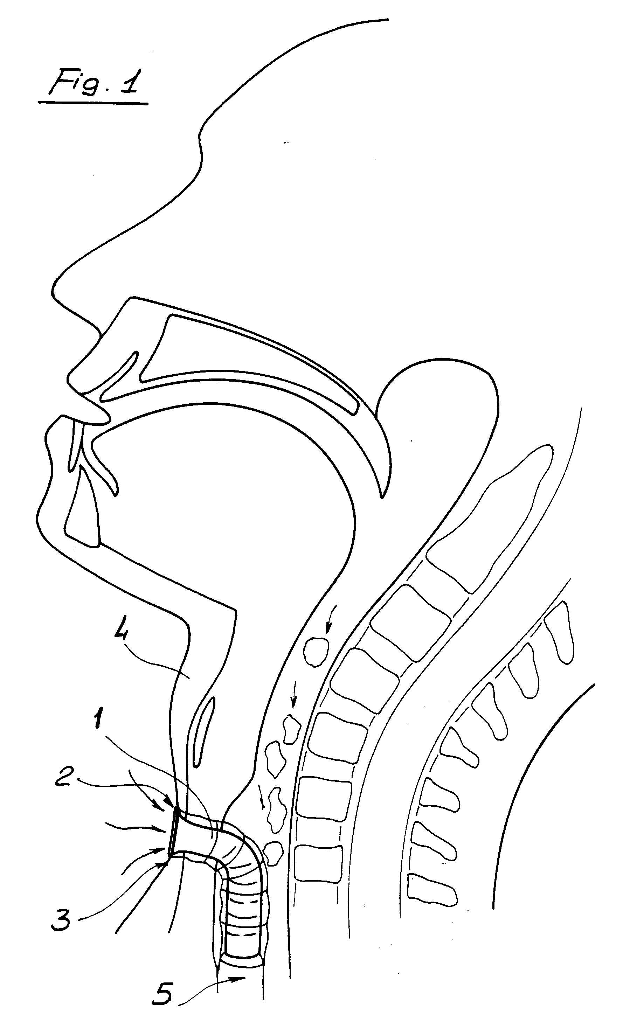

Referring to Fig. 1, patients who undergo a total laryngectomy, using the technique currently adopted, have a cannula 1 inserted in the front opening 2 of the trachea in the skin 3 of the throat 4 (tracheostoma).

In this case of tracheostoma, the cannula 1 maintains the shape for a certain time, permitting only respiration but not phonation, thus compromising normal recovery of the speech function, while deglutition is restored to normality.

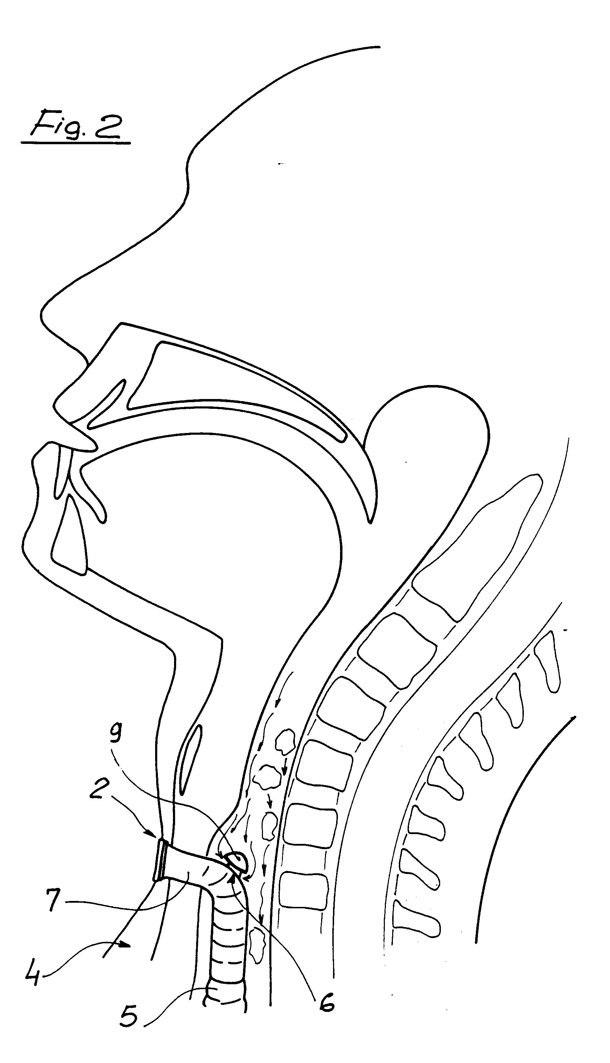

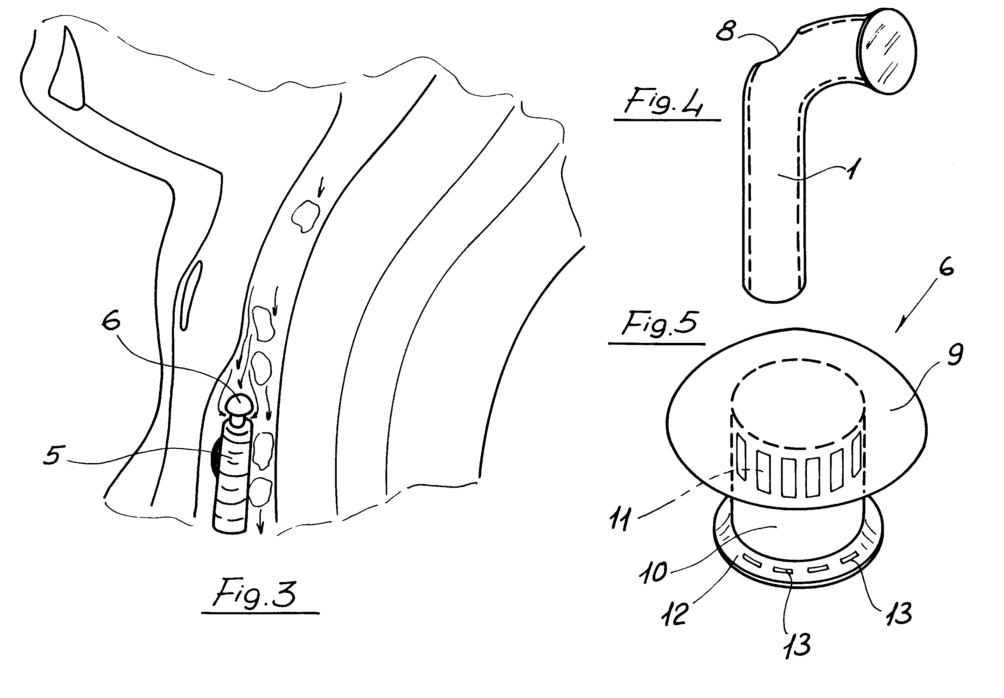

Referring to Fig. 2, to return to a normal condition, reincorporating once again the functions of deglutition, phonation and respiration at a lower pharyngeal level, the phonation valve 6 of this invention is applied to the upper surface 7 of the trachea 5 which emerges at the throat 4 by a forward rotation of its upper opening 2. The cannula 1 (referring to fig. 4) has a window 8 in the anteriad portion which permits the air to pass from the lungs to the phonation valve 6 inserted in the trachea and in contact with the hypopharynx.

Referring to Fig. 5, said valve 6 comprises a protecting cap 9 overlying a drum 10 provided with some slots 11 through which air is passing.

At the base of drum 10 there is a collar 12 provided with radial slots 13 allowing infiltration of the connective tissue and subsequent final sealing and cicatrization to the stump of the trachea.

Referring to fig. 3, the aforesaid valve 6 can also be applied to the upper extremity of the straight trachea 5 left after total laryngectomy if the surgeon has made a tracheostoma with an opening in the side front wall of the throat, thus restoring the functions of respiration and deglutition in an optimal fashion.

In the latter, as is shown in the drawing referred to, the tracheostoma can be eliminated thus also eliminating the embarassment which is a typical condition in patients who have undergone laryngectomies.

However, it must be emphasised that the tracheostoma should be maintained, covering the frontal aperture in some appropriate way, in order to guarantee the respiratory function should any problems arise with the valve.

The valve of this claim weighs only a few grams, is approximately 2 cm wide and is made from a biocompatible anti-allergic material (for example Dacron, Teflon or a similar material) and at least the protective cap 9 should preferably be made of material which is opaque to x-rays.

This description demonstrates the advantages obtained from the use of the phonation valve of this invention, one of the most important being the almost complete recovery of the speech function and total recovery of the patient from the psychological point of view.

Finally, it must be emphasised that the detailed description of this embodiment in no way limits the scope of the invention, since it must be understood that numerous modifications, additions, variations or substitutions of the components can be made to this invention while still remaining within the scope of protection as defined in the attached claims.

## Claims

1. Phonation valve (6) for patients who have undergone laryngectomy, adapted to be applied at the point of interconnection of the trachea (5) and the pharynx allowing recovery of the normal functions of respiration, reincorporating the functions of deglutition, phonation and respiration at the level of the lower pharynx, characterised in that it comprises a drum (10) provided with slots (11) for the passage of air, protected by an overlying protective cap (9) with a convex conformation, allowing air to flow through the slots (11) of the drum (10) and at the same time allowing downward passage of food and liquids during deglutition but substantially preventing food and liquids from entering the trachea.

2. Valve, according to Claim 1, characterised in that the drum (10) comprises a base rim (12) provided with lateral slots (13) , so as to permit infiltration of the connective tissue and its subsequent cicatrising and bonding to the residual stump of the trachea (5).

3. Valve, according to Claim 1 characterised in that it is made from biocompatible anti-allergic materials and that preferably, at least the protective cap (9) is made from materials which are opaque to x-rays.

## Revendications

1. Valve de phonation (6) pour patients ayant subi une laryngectomie, susceptible d'être appliquée au point d'interconnexion de la trachée (5) et du pharynx et permettant la récupération des fonctions normales de la respiration, la reconstitution des fonctions de déglutition, de phonation et de respiration au niveau de pharynx inférieur, caractérisée en ce qu'elle comporte un tambour (10) muni de fentes (11) pour le passage de l'air, protégé par un couvercle de protection surplombant (9) à conformation convexe, permettant à l'air de s'écouler à travers les fentes (11) du tambour (10) et, en même temps, permettant la descente d'aliments et de liquides durant la déglutition mais empêchant, pour l'essentiel, que les aliments et les liquides ne pénètrent dans la trachée.

2. Valve selon la revendication 1, caractérisée en ce que le tambour (10) comporte un rebord de base muni de fentes latérales (13), de façon à permettre l'infiltration du tissu conjonctif et sa cicatrisation ainsi que sa liaison ultérieures au moignon résiduel de la tranchée (5).

**3.** Valve selon la revendication 1, caractérisée en ce qu'elle est réalisée en matériaux anti-allergiques et biocompatibles et que, de préférence, au moins le couvercle de protection (9) est réalisé en matériaux opaques aux rayons X.

**Patentansprüche**

**1.** Stimmbildungsventil (6) für Patienten, welche einer Laryngektomie unterzogen wurden, wobei dieses Stimmbildungsventil dazu abgebildet ist, an einer Verbindungsstelle der Trachea (3) und des Pharynx angebracht zu werden und ein Wederherstellan der normalen Funktion der Respiration unter Wiedereinverleibung der Funktionen Deglutition, Stimmbildung und Respiration in einem unteren Pharynx-Bereich gewährleisten,
dadurch gekennzeichnet,
daß das Stimmbildungsventil eine mit Schlitzen (11) versehene Trommel (10) zum Durchlassen von Luft aufweist, weiche von einer überdekkenden Schutzkappe (9) mit einer konvexen Form geschützt wird, welche eine Luftströmung durch die Schlitze der Trommel (10) erlaubt und zugleich ein Durchlassen von Speisen und Getränken während der Deglutition gestattet, jedoch zugleich das Eindringen von Speisen und Getränken in die Trachea im wesentlichen verhindert.

**2.** Stimmbildungsventil nach Anspruch 1,
dadurch gekennzeichnet,
daß die Trommel (10) elnen Fußrand (12) aufweist, welcher mit seitlichen Schlitzen (13) versehen ist, welche das Eindringen von Verbindungsgewebe und die nachfolgende Vernarbung und damit Verbindung mit dem verbliebenen Stumpf der Trachea (5) ermöglichen.

**3.** Stimmbildungsventil nach Anspruch 1,
dadurch gekennzeichnet,
daß dieses aus biokompatiblem und antiallergenem Material hergestellt ist und vorzugsweise zumindest die Schutzkappe (9) aus für Röntgenstrahlen undurchlässigem Material hergestellt ist.

Fig. 1

_Fig. 2_

Fig.3

Fig.4

Fig.5

EP 0 279 484 B1